# EUROPEAN PATENT SPECIFICATION

(11) **EP 3 083 965 B1**
(45) Date of publication and mention of the grant of the patent: **10.07.2019**
(21) Application number: 14811909.2
(22) Date of filing: 12.12.2014
(51) Int. Cl.: C07K 14/245, C12N 15/70, C12P 21/00, C12N 15/64, C12N 15/68

(54) **A PROCESS FOR PRODUCTION OF A PROTEIN OF INTEREST IN A MICROBIAL HOST ORGANISM**
VERFAHREN ZUR PRODUKTION VON BESTIMMTEN PROTEINEN IN EINEM MIKROBIELLEN WIRTSORGANISMUS
PROCÉDÉ DE PRODUCTION D'UNE PROTÉINE D'INTÉRÊT CHEZ UN ORGANISME HÔTE MICROBIEN

(30) Priority: 20.12.2013 EP 13198797
(43) Date of publication of application: 26.10.2016
(73) Proprietor: BASF SE, 67056 Ludwigshafen am Rhein (DE)
(72) Inventor: JENEWEIN, Stefan, 67434 Neustadt (DE); FELLE, Max Fabian, 67346 Speyer (DE); REYELT, Jan, 69221 Dossenheim (DE)
(74) Representative: BASF IP Association
(86) International application number: PCT/EP2014/077604
(87) International publication number: WO 2015/091280

(56) References cited:
- WO-A1-2012/123429
- WO-A2-2014/037440
- US-A1- 2004 234 984
- Laszlo Janosi ET AL: "Evidence for in vivo ribosome recycling, the fourth step in protein biosynthesis", The EMBO Journal, 15 February 1998 (1998-02-15), pages 1141-1151, XP55165143, Chichester, UK DOI: 10.1093/emboj/17.4.1141 Retrieved from the Internet: URL:http://onlinelibrary.wiley.com/doi/10. 1093/emboj/17.4.1141/abstract

## Description

The present invention relates to a novel process for recombinant production of a protein of interest in a microbial host organism without the need of antibiotic selection pressure for the presence and maintenance of vector DNA. It also embraces a transformed host cell, in which a chromosomal frr gene has been inactivated and which comprises a vector containing a functional frr gene.

### State of the Art

The stable maintenance of a vector, particularly at high copy number, is important for the preparation of DNA and the protein expressed from it. A cloning vector, such as a plasmid, comprising cloned genes and having been introduced into a host such as a bacteria is easily lost during cultivation of the bacteria. This is because of uncontrolled distribution of the vector to the daughter cells upon cell division. Also, the vector is a metabolic burden to the bacterial host. Bacteria having lost the plasmid generally grow better and take on in numbers compared to the ones harbouring the plasmid.

Accumulation of plasmid-free segregants represents a problem for basic replicon plasmids that are used for cloning and expression purposes, since they do not carry any gene system for an ordered distribution to the host daughter cells. This represents a major industrial problem for maintenance of expression plasmids in the host bacterium during large-scale cultivation. A number of approaches have been tried in order to overcome segregational instability of cloning plasmids.

The use of some antibiotic resistance gene inserted into the vector and addition of the corresponding antibiotic to the growth medium represents the most conventional selection pressure for plasmid maintenance. In addition to the economic cost of the antibiotic, a potential environmental hazard from both the antibiotic itself and the resistance gene in the industrial waste is obvious. Appearance of plasmid-free segregants is not prevented totally, since the concentration of the antibiotic used for the plasmid selection often decreases during long-term cultivation as a result of dilution and/or enzymatic degradation.

US 2004/0234984 relates to a method for deletion of antibiotic resistance and/or creation of vector stabilisation in a host cell. According to the present invention a new vector is produced that gives a total selection for host cells that carry this vector. This property is obtained by deleting the chromosomal copy of an essential gene and replacing it into the plasmid. As a result only plasmid carrying cells can grow, making the host totally dependent on the plasmid. Preferably a small gene such as the gene infA is used. The gene infA codes for the translation-initiation factor 1 (IF1) which is a small intracellular and essential factor for cell viability. The plasmid does not contain any antibiotic gene and antibiotic selection is thus not necessary, providing considerable advantages during cultivation and elimination of significant environmental concerns. The method is especially advantageous for stabilisation by means of vectors in plant bacteria for transgenic plants to be set out in greenhouses and in nature, where no elimination of the host is envisaged.

WO 2012/123429 A1 is concerned with a filamentous fungal host cell deficient in an essential gene, comprising a vector, said vector comprising at least said essential gene and an autonomous replication sequence. In this respect the document also relates to a method for the production of a biological compound of interest comprising culturing the vector-host system or the host cell under conditions conducive to the production of the biological compound of interest.

Janosi et al. (Proc. Natl. Acad. Sci. USA Vol. 91, pp4249-4253, May 1994) describe that the ribosome releasing factor - product of the frr gene in E. coli - is essential for bacterial growth.

Weixlbaumer et al. (Nature Structural and Molecular Biology, Vol. 14, Number 8, pages 733-737, August 2007) describe the crystal structure of the ribosome recycling factor bound to a ribosome.

The task of this invention is to provide a process for recombinant production of a protein of interest in a microbial host organism without the need of antibiotic selection pressure for the presence and maintenance of vector DNA. A further task of the invention is to couple the growth of the host organism to the presence of the vector in the host organism in a very sensitive way with the aim to stop growth of those host organisms immediately which have lost their vector.

### Description of the Invention

One embodiment of the invention is a process for production of a protein of interest in a bacterial host organism comprising the steps of:
a) providing a bacterial host organism having an inactivated chromosomal frr gene and carrying a helper plasmid, the helper plasmid comprising a functional frr gene and a marker gene,
b) transforming the host organism with a vector comprising a gene for the protein of interest and a functional frr gene and no antibiotic resistance gene,,
c) culturing the transformed host organism obtained in b) under conditions allowing the expression of the gene of interest in the host organism and
d) isolating the protein of interest,
wherein the host organism is selected for the absence of the helper plasmid after transformation with the vector

The inventions deals with the recombinant production of proteins in microorganisms.

For the disclosed invention proteins of interest (Pol), are amino acid chains in which from 10 to about 1000, for example 20 to 700 and in particular 50 to 500, amino acids are linked via peptide bonds. Peptides may be composed of any alpha-amino acids, in particular the proteinogenic amino acids.

These Pol can be enzymes or non-enzymatic proteins. Examples of enzymes are lipases, dehydrogenases, oxidases, proteases. Examples of non-enzymatic proteins are such with antimicrobial activity, specific binding to certain surfaces, nucleating properties in crystallization processes and particle formation, control of crystal structures, binding of metals or metal ions, surfactant properties, emulsifying properties, foam-stabilizing properties, influencing cellular adsorption.
A gene for a protein of interest (Pol gene) is a polynucleotide sequence coding for the Pol. Such a polynucleotide sequence can be isolated from genomes by known techniques or synthesized by DNA synthesis methods. The term Pol gene is understood in a broad sense meaning that it can - in addition to the part coding for the Pol - encompass also regulatory parts such as promoters, which are associated with the expression of the Pol in its original genetic background.

A microbial host system suitable for the invention is in principle any organisms that enable the nucleic acids according to the invention to be expressed. Host organisms mean bacteria.

Non-limiting examples of prokaryotic expression organisms are Escherichia coli, Bacillus subtilis, Bacillus megaterium, Corynebacterium glutamicum, and others.

The invention relates to expression constructs comprising a nucleic acid sequence coding for a protein of interest under genetic control by regulatory nucleic acid sequences, and to vectors comprising at least one of said expression constructs. Such constructs according to the invention preferably comprise a promoter 5' upstream of the particular coding sequence, and a terminator sequence 3' downstream, and optionally further common regulatory elements which in each case are operatively linked to the coding sequence.

"Operative linkage" means the sequential arrangement of promoter, coding sequence, terminator and optionally further regulatory elements in such a way that each of said regulatory elements can carry out its intended function during expression of the coding sequence. Examples of operatively linkable sequences are targeting sequences and also enhancers, polyadenylation signals and the like. Further regulatory elements comprise selectable markers, amplification signals, origins of replication and the like. Suitable regulatory sequences are described, for example, in Goeddel, Gene Expression Technology: Methods in Enzymology 185, Academic Press, San Diego, California(1990).

The natural regulatory sequence may still be present upstream of the actual structural gene, in addition to the artificial regulatory sequences. This natural regulation may optionally be switched off by genetic modification, thereby increasing or reducing expression of the genes. However, the gene construct may also have a simpler structure, i.e. no additional regulatory signals are inserted upstream of the structure gene, and the natural promoter with its regulation is not removed. Instead, the natural regulatory sequence is mutated in such a way that regulation no longer takes place and gene expression is increased or reduced. The gene construct may comprise one or more copies of the nucleic acid sequences.

Examples of usable promoters are: cos, tac, trp, tet, trp-tet, Ipp, lac, Ipp-lac, laclq, T7, T5, T3, gal, trc, ara, SP6, lambda-PR or in lambda-PL promoter, which are advantageously used in gram-negative bacteria; and also the gram-positive promoters amy and SPO.sub.2. Particular preference is given to using inducible promoters such as, for example, light- and particularly temperature-inducible promoters such as the P.sub.rP.sub.I promoter. In principle, any natural promoters with their regulatory sequences may be used. In addition, synthetic promoters may also be used advantageously.

Said regulatory sequences are intended to enable the nucleic acid sequences and the proteins to be expressed in a specific manner. Depending on the host organism, this may mean that the gene is expressed or overexpressed only after induction or that it is expressed and/or overexpressed immediately, for example.

Preference is given here to the regulatory sequences or factors being able to positively influence and thereby increase or reduce expression. Thus it is possible for the regulatory elements to be enhanced advantageously at the transcriptional level by using strong transcription signals such as promoters and/or "enhancers". In addition, however, it is also possible to enhance translation, for example by improving the stability of mRNA.

An expression cassette is prepared by fusing a suitable promoter to a suitable coding nucleotide sequence and to a termination or polyadenylation signal. For this purpose, familiar recombination and cloning techniques are used, as described, for example, in T. Maniatis, E. F. Fritsch and J. Sambrook, Molecular Cloning: A Laboratory Manual, Cold Spring Harbor Laboratory, Cold Spring Harbor, N.Y. (1989) and in T. J. Silhavy, M. L. Berman and L. W. Enquist, Experiments with Gene Fusions, Cold Spring Harbor Laboratory, Cold Spring Harbor, N.Y. (1984) and in Ausubel, F. M. et al., Current Protocols in Molecular Biology, Greene Publishing Assoc. and Wiley Interscience (1987).

To express the recombinant nucleic acid construct or gene construct in a suitable host organism, it is advantageously inserted into a host-specific vector which enables the genes to be optimally expressed in the host. Vectors are well known to the skilled worker and can be found, for example, in "Cloning Vectors" (Pouwels P. H. et al., eds., Elsevier, Amsterdam-New York-Oxford, 1985). Vectors are understood to include in addition to plasmids also any other vectors known to the skilled worker, such as phages, transposons, IS elements, cosmids, and linear or circular DNA, for example.

Examples of suitable expression vectors which may be mentioned are: Common fusion expression vectors such as pGEX (Pharmacia Biotech Inc; Smith, D. B. and Johnson, K. S. (1988) Gene 67:31-40), pMAL (New England Biolabs, Beverly, Mass.) and pRIT 5 (Pharmacia, Piscataway, N.J.), in which glutathione S transferase (GST), maltose E-binding protein and protein A, respectively, are fused to the recombinant target protein.

Non-fusion protein expression vectors such as pTrc (Amann et al., (1988) Gene 69:301-315) and pET 11d (Studier et al. Gene Expression Technology: Methods in Enzymology 185, Academic Press, San Diego, Calif. (1990) 60-89).
Ribosome Recycling Factor (frr) is a protein found in bacterial cells as well as eukaryotic organelles, specifically mitochondria and chloroplasts. It functions to recycle ribosomes after completion of protein synthesis. Recent evidence suggests frr may accomplish the recycling of ribosomes by splitting ribosomes into subunits, thereby releasing the bound mRNA. In Bacteria (specifically Escherichia coli), loss of the gene frr encoding Ribosome Recycling Factor is deleterious.

Functional frr gene means a gene that can complement the function of an inactive or absent frr gene in an organism. This can be the homologous frr gene of the microbial host organism or a heterologous frr gene or a mutated frr gene.

Inactive or inactivated gene especially inactivated frr gene means a gene, especially frr gene that is not expressed in a functional gene product, especially the Ribosome Recycling Factor. An inactivation of the frr gene can be effected in different ways, i.e. by deleting completely or partly the frr gene in the chromosome or by inserting into the frr gene additional nucleotides or polynucleotides in order to destroy the reading frame of the gene. Another possibility to inactivate a gene is to introduce stop codons into the reading frame or to delete control elements of the gene such as promotor regions. There are well-known procedures in the art for the inactivation of genes by knock-out or insertional mutagenesis.

The frr gene of E.coli codes for a 185aa protein. The polypeptide sequence can be found in NCBI (Protein) GI: 16128165.
The vector used for the transformation of the microbial host organism having a functional frr gene has no antibiotic resistance gene, because an antibiotic resistance is not necessary for the selection of host organisms carrying the vector.

Transforming means the introduction of DNA such as a vector or a helper plasmid into the microbial host organism. It can be performed by known techniques such as Calciumphosphate precipitation or electroporation.

Recombineering in the microorganism E.coli can be performed as described by Zhang et al. (Nature Genetics, 20, 123-128, 1998).

The recombinant microorganism may be cultured and fermented according to known methods. For example, bacteria may be propagated in TB or LB medium and at from 20 to 40° C and pH 6 to 9. Suitable culturing conditions are described in detail, for example, in T. Maniatis, E. F. Fritsch and J. Sambrook, Molecular Cloning: A Laboratory Manual, Cold Spring Harbor Laboratory, Cold Spring Harbor, N.Y. (1989).

Unless the proteins of interest (Pol) are secreted into the culture medium, the cells are then disrupted and the product is recovered from the lysate by known protein isolation methods. The cells may optionally be disrupted by high-frequency ultrasound, by high pressure, for example in a French pressure cell, by osmolysis, by the action of detergents, lytic enzymes or organic solvents, by homogenizers, or by combining a plurality of the methods listed.

The Pol may be purified using known, chromatographic methods such as molecular sieve chromatography (gel filtration) such as Q-Sepharose chromatography, ion exchange chromatography, and hydrophobic chromatography, and by other common methods such as ultrafiltration, crystallization, salting out, dialysis and native gel electrophoresis. Suitable methods are described, for example, in Cooper, F. G., Biochemische Arbeitsmethoden [original title: The Tools of Biochemistry], Verlag Walter de Gruyter, Berlin, New York or in Scopes, R., Protein Purification, Springer Verlag, New York, Heidelberg, Berlin.

Furthermore, the recombinant Pol may be isolated by using vector systems or oligonucleotides which extend the cDNA by particular nucleotide sequences and therefore code for modified polypeptides or fusion proteins which are used for simpler purification, for example. Examples of suitable modifications of this kind are "tags" acting as anchors, for example the modification known as hexa-histidine anchor, or epitopes that can be recognized as antigens by antibodies (described, for example, in Harlow, E. and Lane, D., 1988, Antibodies: A Laboratory Manual, Cold Spring Harbor (N.Y.) Press). These anchors may be used for fixing the proteins to a solid support such as, for example, a polymer matrix which may be introduced, for example, into a chromatographic column or may be used on a microtiter plate or another support.

At the same time, these anchors may also be used for recognizing the proteins. The proteins may moreover be recognized by using common markers such as fluorescent dyes, enzyme markers forming a detectable reaction product upon reaction of a substrate, or radioactive markers, either alone or in combination with said anchors, in order to derivatize the proteins.

An advantage of the invention is that the host organism having an inactivated chromosomal frr gene is viable because of the complementation by the helper plasmid.

When the new vector carrying the gene of interest and a functional frr gene is used to transform this host, the host is carrying immediately after transformation two different vectors, (i) the helper plasmid and (ii) the vector carrying the gene of interest, with both vectors being able to the complementation of the inactivated chromosomal frr gene. As the helper plasmid carries in addition to the functional frr gene also a marker gene, which allows to screen for the absence or presence of the marker, it is possible to select or counterselect for or against this marker. So it is possible to select transformed host organisms carrying only the vector a) and no helper plasmid anymore.

Another embodiment of the invention is a transformed microbial host cell, characterized in that its chromosomal gene for the frr is inactivated and in that it comprises a vector comprising at least one copy of a functional frr gene together with a gene of interest and in that it does not comprise any gene for antibiotic resistance.

Further embodiments of the invention are disclosed in the claims.

## Claims

1. A process for production of a protein of interest in a bacterial host organism comprising the steps of:
a) providing a bacterial host organism having an inactivated chromosomal frr gene and carrying a helper plasmid,
the helper plasmid comprising a functional frr gene and a marker gene ,
b) transforming the host organism with a vector comprising a gene for the protein of interest and a functional frr gene and no antibiotic resistance gene,
c) culturing the transformed host organism obtained in b) under conditions allowing the expression of the gene of interest in the host organism and
d) isolating the protein of interest,
wherein the host organism is selected for the absence of the helper plasmid after transformation with the vector.

2. A process according to claim 1, wherein the frr gene upon the vector is homologous to the host organism.

3. A process according to claim 1 or 2, wherein the host organism is Escherichia coli, Bacillus subtilis, Bacillus megaterium or Corynebacterium glutamicum.

4. A process according to any of the previous claims, wherein the inactivated chromosomal frr gene of the host organism is effected by gene deletion.

## Patentansprüche

1. Verfahren zur Herstellung eines Proteins von Interesse in einem bakteriellen Wirtsorganismus, welches die folgenden Schritte umfasst:
a) die Bereitstellung eines bakteriellen Wirtsorganismus, der ein inaktiviertes chromosomales frr-Gen aufweist und ein Helferplasmid trägt,
wobei das Helferplasmid ein funktionelles frr-Gen und ein Markergen umfasst,
b) das Transformieren des Wirtsorganismus mit einem ein Gen für das Protein von Interesse und ein funktionelles frr-Gen und kein Gen für Antibiotikaresistenz umfassenden Vektors,
c) das Kultivieren des in b) erhaltenen transformierten Wirtsorganismus unter Bedingungen, die die Expression des Gens von Interesse in den Wirtsorganismus erlauben, und
d) das Isolieren des Proteins von Interesse,
wobei der Wirtsorganismus auf Abwesenheit des Helferplasmids nach Transformation mit dem Vektor selektiert wird.

2. Verfahren nach Anspruch 1, wobei das frr-Gen auf dem Vektor homolog mit dem Wirtsorganismus ist.

3. Verfahren nach Anspruch 1 oder 2, wobei es sich bei dem Wirtsorganismus um Escherichia coli, Bacillus subtilis, Bacillus megaterium oder Corynebacterium glutamicum handelt.

4. Verfahren nach einem der vorhergehenden Ansprüche, wobei die Inaktivierung des chromosomalen frr-Gens des Wirtsorganismus durch Gendeletion erfolgt.

## Revendications

1. Procédé pour la production d'une protéine d'intérêt dans un organisme hôte bactérien comprenant les étapes de :
a) mise à disposition d'un organisme hôte bactérien possédant un gène frr chromosomique inactivé et portant un plasmide auxiliaire, le plasmide auxiliaire comprenant un gène frr fonctionnel et un gène marqueur,
b) transformation de l'organisme hôte par un véhicule comprenant un gène pour la protéine d'intérêt et un gène frr fonctionnel et aucun gène de résistance aux antibiotiques,
c) culture de l'organisme hôte transformé obtenu dans l'étape b) dans des conditions permettant l'expression du gène d'intérêt dans l'organisme hôte et
d) isolement de la protéine d'intérêt,
l'organisme hôte étant choisi en raison de l'absence du plasmide auxiliaire après transformation par le véhicule.

2. Procédé selon la revendication 1, le gène frr sur le vecteur étant homologue à l'organisme hôte.

3. Procédé selon la revendication 1 ou 2, l'organisme hôte étant Escherichia coli, Bacillus subtilis, Bacillus megaterium ou Corynebacterium glutamicum.

4. Procédé selon l'une quelconque des revendications précédentes, le gène frr chromosomique inactivé de l'organisme hôte étant obtenu par délétion de gène.
